# EUROPEAN PATENT SPECIFICATION

(11) **EP 3 237 022 B1**
(45) Date of publication and mention of the grant of the patent: **04.04.2018**
(21) Application number: 15794316.8
(22) Date of filing: 06.09.2015
(51) Int. Cl.: A61L 9/03, A61L 9/22, H01T 23/00, F24F 3/16, C01B 15/027, C25B 1/30

(54) **A METHOD AND DEVICE FOR GENERATION OF HYDROGEN PEROXIDE**
VERFAHREN UND VORRICHTUNG ZUR ERZEUGUNG VON WASSERSTOFFPEROXID
PROCÉDÉ ET DISPOSITIF DE GÉNÉRATION DE PEROXYDE D'HYDROGÈNE

(30) Priority: 23.12.2014 US 201414581294
(43) Date of publication of application: 01.11.2017
(73) Proprietor: Oxypro Ltd., Katzrin 1290000 (IL)
(72) Inventor: RISKIN, Yefim, 1290000 Katzrin (IL)
(74) Representative: Petruzziello, Aldo
(86) International application number: PCT/IL2015/050897
(87) International publication number: WO 2016/103246

(56) References cited:
- EP-A1- 2 404 621
- WO-A2-96/32175
- WO-A2-2007/130852
- DE-A1-102009 017 807
- US-A- 3 440 800
- US-A- 5 380 355

## Description

### FIELD OF THE INVENTION

This invention relates to methods and devices for the generation of hydrogen peroxide operating on the principle of conveying air-liquid or vapor flow through a corona discharge zone in air.

### BACKGROUND OF THE INVENTION

CN 1011569515 discloses a non-equilibrated plasma type spray sterilization disinfectant generating device, consisting of a main unit and a plasma spray head. A high voltage potential difference between metal electrodes in the plasma spray head is utilized to form a corona discharge where the distance between the metal electrodes is minimal. The corona discharge is driven by an air-water mixture generated by an atomization nozzle, so as to quickly slide downstream along an electrode surface and form a pulse type sliding arc discharging non-equilibrated plasma on the electrode surface. An air-water mixture containing hydrogen peroxide, ozone, oxyhydrogen free radical, oxygen free radical, and other oxidizable particles is produced and sprayed from the insulator casing to form air-water spray sterilization disinfectant.

It is also known to produce the air-liquid spray using an air compressor which conveys air at a high speed (50 - 100 m/sec) through a corona discharge zone.

The flow conveyed through the corona discharge zone contains hydrogen peroxide (H₂O₂) and ozone (O₃) which are delivered to the object being disinfected. A drawback of such a method is that part of the ozone is dissolved in the liquid, while the remainder is present in the airflow.

Also known is an approach in which the air-liquid spray is again produced by means of an air compressor and conveyed through a corona discharge zone, but is thereafter separated into liquid hydrogen peroxide and air which contains undissolved ozone in the liquid.

In order to remove the ozone, the air may be conveyed through an activated carbon filter.

A common disadvantage of the previous state of the art is its limited field of application which stems from the need for an air compressor.

EP 2 404 621 discloses an ionization device for decontaminating a contaminated medium with oxygen ions enriched air and comprises an insulating housing with an air supply, an air outlet and a voltage source, and a ventilator with wings arranged in the housing, whose ends are coated with an electrically conductive metal to form as electrodes. The electrodes at the end of the wings interact with counter electrodes surrounding the ventilator, which are arranged in the housing. In use, the ventilator is rotated while introducing the air through the air supply, ionizing the air by the device, and removing the air from the apparatus through the air outlet and introducing it into the medium to be treated.

US Patent No. 5,380,455 discloses a waste airstream decontamination unit having a series of grounded electrodes extending vertically from rotating shafts mounted transversely across a decontamination chamber. Corresponding upper and lower positive electrodes extend toward the rotating grounded electrodes in an angular relationship. Rotation of the shafts causes the charged electrodes to produce and sustain a repeating sequence of arcs which ionize and control the airborne contaminants which are passed through the chamber,

US Patent No. 3,440,800 discloses a device for cleaning exhaust gases including a housing with an input end and an output end, a permeable cylindrical collecting electrode located in the housing and defining an ionization space, a shaft located in and coaxial with the collecting electrode, and a plurality of ionizing electrodes projecting radially outward from the shaft. An electrostatic potential is maintained between the collecting and ionizing electrodes, said flow-path means further including a turbine chamber preceding said housing, said chamber being equipped with a gas inlet connection and with a hot gas turbine including a turbine rotor engaging said shaft for rotating said shaft about its axis, gas tube input means centrally positioned with respect to said collecting electrode so that exhaust gases enter said ionizing space and pass with a radial component of motion through said permeable collecting electrode, said gas tube input means opening into said turbine chamber and aperture means of variable cross-section surrounding said input tube for the introduction of fresh air into said ionization space.

### SUMMARY OF THE INVENTION

An object of the present invention is therefore to provide a method and device for generating hydrogen peroxide which address disadvantages of the prior art.

This object is in the first place achieved by generating cold vapor, which is used as an air-liquid flow, using the well-known method of ultrasonic generation in water, thus avoiding use of the costly compressor.

Unlike the air-liquid flow generation by means of an air compressor which is related to jet technologies, cold vapor generation can be related to "cloud technologies", since the cloud vapor is generated under normal pressure when the vapor "cloud" remains immobile unless an external source is used to generate a pressure fall. A fan is generally used as such a source.

The proposed method is based on the use of a hollow rotor which performs three functions simultaneously.
1. Operates or drives one of the electrodes used to generate a corona discharge zone;
2. Generates a discharge zone for the cold vapor transfer into the corona discharge zone;
3. Operates as a liquid and air separator which uses centrifugal force produced by the rotor rotation.

The rotor or at least a part of it is made of a conducting material.

The other electrode which is coated with insulation is mounded inside the rotor cavity.

Power from a high voltage AC source is applied to the rotor from one of the terminals via capacitive coupling while the second terminal is directly connected to the second electrode. In this way a straight segment of corona discharge is generated between the rotor and the second electrode insulator.

The distance between the inner diameter of the rotor and the outer diameter of the second electrode insulator is determined from a consideration of the required corona discharge current produced at a preset magnitude of the high AC voltage.

The rotor shaped as a centrifugal fan rotor is rotated around the second electrode. The corona discharge zone is rotated too.

As mentioned before the cold vapor is drawn into and rotates within the corona discharge zone.

The rotational velocity of the rotor and the corona discharge zone is much higher than that of the cold vapor because of the strong impact of the corona discharge on the vapor. It is subjected to a strong influence of the corona discharge.

Vapor separation to liquid and air using centrifugal force produced by the rotor is attained by changing by 90° the direction of the cold vapor transmission through the straight segment of corona discharge between the electrodes. The above angle may change slightly depending on the electrodes configuration.

One of the advantages of the proposed method of H₂O₂ generation is the increase of the H₂O₂ concentration when hydrogen peroxide produced at the generator outlet in a liquid form is used to generate the vapor entering the generator. To do this the generator outlet is connected to a reservoir of liquid that is intended to be converted into vapor.

Actually the concentration is increased due to the proportional increase of the generator operation time.

A device according to the invention for generating hydrogen peroxide (H₂O₂) comprises:
A cylindrical body with a cold vapor inlet and an air and H₂O₂ outlets from the body, a bypass air channel, stator and rotor plates of air capacitor, a motor with a rotor plate of the air capacitor fastened to its axis and a rotor which together with the motor and the body form a centrifugal fan, an insulator with an electrode mounted inside the rotor cavity and a high AC voltage supply.

### BRIEF DESCRIPTION OF THE DRAWINGS

In order to understand the invention and to see how it may be carried out in practice, embodiments will now be described, by way of non-limiting example only, with reference to the accompanying drawings, in which:
**Fig. 1** is a schematic representation of a device for generating hydrogen peroxide according to the invention; and
**Fig. 2** is a partial cross-section through line A-A in Fig. 1.

### DETAILED DESCRIPTION OF EMBODIMENTS

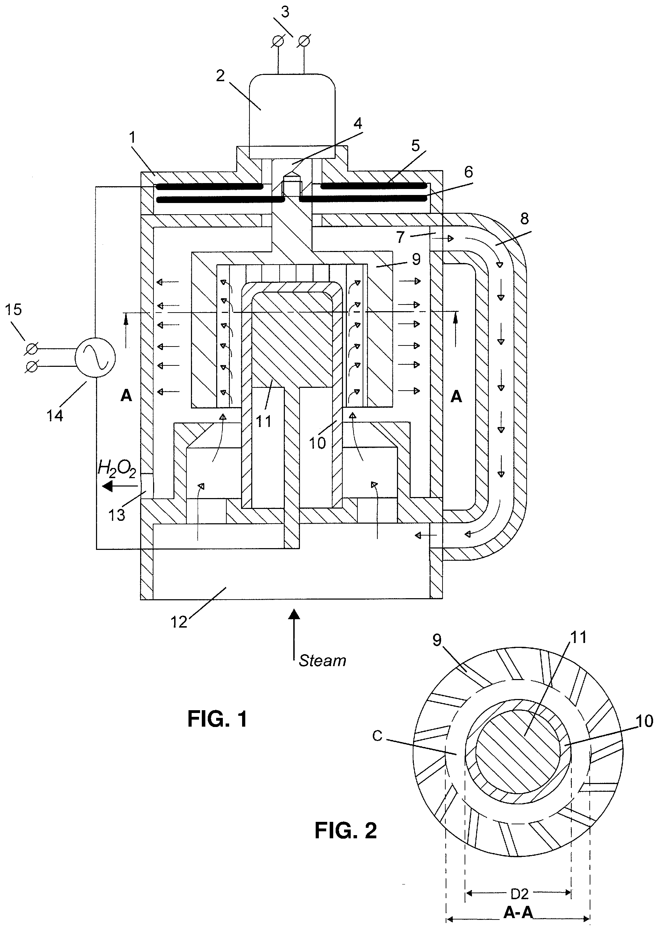

Referring to Fig. 1 there is shown a device for generating hydrogen peroxide comprising a housing 1 toward an upper end of which there are mounted an electric motor 2 having power supply terminals 3 and an axis 4. An air capacitor is formed of an annular stator plate 5 which is supported toward the upper end of the housing and a rotor plate 6 (constituting a first electrode), which together with a hollow rotor 9 are attached to the motor axis 4. Also provided toward the upper end of the housing is an air outlet 7. An insulator 10 containing therein an electrode 11 constituting a second electrode is fixedly mounted inside the hollow rotor 9. A cold vapor inlet 12 and an H₂O₂ outlet 13 are formed in a lower part of the housing 1. The air outlet 7 is connected to the cold vapor inlet 12 via a bypass channel 8. A high voltage AC supply 14 fed by input terminals 15 has outputs coupled to the first electrode constituted by the rotor plate 6 via the stator plate 5 of the air capacitor and to the second electrode 11.

The device operates as follows. As power is applied to the terminals 3 of the motor 2, the motor axis 4 rotates together with the rotor plate 6 of the air capacitor and the rotor 9, both of which are fastened to the motor axis 4. As power is applied to the terminals 15 of the high AC voltage supply 14, the stator plate 5 of the air capacitor and the second electrode 11 are energized. In this way the high voltage AC is applied via the air capacitor to the rotor 9 and the second electrode 11 enclosed within the insulator 10.

A barrier corona discharge zone shown as C in Fig. 2 is generated between the insulator 10 and the rotor 9 and is rotated together with the rotor 9 relative to the fixed insulator 10. At the same time, a low pressure zone created between the rotor 9 and the insulator 10 sucks in the cold vapor from the vapor inlet 12 into the corona discharge zone C. The vapor that passes through the corona discharge zone turns to hydrogen peroxide (H₂O₂) and changes its initial flow direction in the zone by 90°.

Also referring to Fig. 2 it is seen that the corona discharge zone C is formed within the annular space between the inner diameter D1 of the rotor 9 and the outer diameter D2 of the insulator 10 surrounding the second electrode 11. The distance between D1 and D2 is determined from a consideration of the required corona discharge current produced at a preset magnitude of the high AC voltage.

Since centrifugal force is generated during rotation of the rotor 9, large groups of liquid molecules composing the vapor are repelled to the cylindrical inner walls of the housing 1 forming drops of H₂O₂ which flow down along the inner walls and are removed from the device via the H₂O₂ outlet 13 in the lower part of the housing 1.

As a result of the liquid separation process described above, air containing a certain amount of vaporous H₂O₂ and ozone O₃ undissolved in the liquid remains in the device. Escape of air containing the above components from the device is undesirable. Therefore the air exiting the air outlet 7 is conveyed via the by-pass channel 8 to the cold vapor inlet 12 which allows for vapor injection into the device 10.

By such means, recycled air circulates within in a closed space inside the device, thus obviating the need for an activated carbon filter and simplifying the design and maintenance of the device.

A prototype of the device constructed using the proposed method has the following specification:

| Insulator material | Glass |
|---|---|
| Distance between the insulator of the first electrode and the second electrode | 1mm |
| The rotor rotation velocity | 6000RPM |
| The AC voltage source amplitude | ± 7 kV |
| The AC voltage source frequency | 40 kHz |
| Vapor consumption | 300 mL/h |
| H₂O₂ concentration at the generator outlet | 100 ppm |

## Claims

1. Device for generating hydrogen peroxide, the device comprising:
a housing (1) having an inlet (12) for air-liquid or vapor supply and separate outlets (13, 7) for H₂O₂ and air outflow,
first and second electrodes (6, 11) positioned to allow the air-liquid or vapor flow, and
a high AC voltage supply (14) connected to the first and second electrodes and configured to generate between the electrodes a corona discharge zone having a straight segment;
wherein one of the first and second electrodes changes the direction of the air-liquid or vapor flow within the straight segment of the corona discharge zone;
**characterized in that:**
the first electrode (6), which changes the direction of the air-liquid or vapor flow, is coupled to a hollow rotor (9),
the second electrode (11) is surrounded by an insulator (10) and is disposed within a cavity of the hollow rotor, and
the H₂O₂ outlet (13) is located in the lower part of the housing (1).

2. The device according to claim 1, wherein at least part of the rotor is made from an electrically conducting material.

3. The device according to claim 2, wherein the high AC voltage supply (14) is connected to the rotor (9) via a capacitive coupling (5, 6).

4. The device according to any one of claims 1 to 3, wherein the rotor (9) is configured to rotate relative to the second electrode (11).

5. The device according to any one of claims 1 to 4, wherein an inner diameter (D1) of the rotor (9) is displaced from an outer diameter of the insulator (D2) by a predetermined gap, a length of which is selected so as to derive a required corona discharge current at a preset magnitude of the high AC voltage.

6. The device according to claim 1, wherein the air outlet and the air-liquid or vapor inflow inlet are connected by a bypass channel.

7. Method for generating hydrogen peroxide (H₂O₂) using the device according to any one of claims 1 to 6, the method comprising:
conveying air-liquid or vapor flow through a corona discharge zone (C) having a straight segment generated by applying high AC voltage (14) between first and second electrodes (6, 11), wherein:
the angle of the flow direction is changed in the course of its passage in the straight segment of the corona discharge zone.

8. The method according to claim 7, wherein one of the electrodes (11) is mounted within a hollow rotor (9), rotation of which forms centrifugal force.

9. The method according to claim 8, wherein at least part of the rotor (9) is made from an electrically conductive material.

10. The method according to claim 7, wherein high AC voltage is connected to the rotor (9) via a capacitive coupling (5, 6).

11. The method according to claim 7, wherein the second electrode (11) is surrounded by an insulator (10) and positioned inside a cavity of the hollow rotor.

12. The method according to claim 11, wherein the rotor (9) is rotated relative to the second electrode (11).

13. The method according to claim 7, wherein an inner diameter (D1) of the hollow rotor is displaced from an outer diameter (D2) of the insulating coating of the second electrode by a predetermined gap, a length of which is selected so as to derive a required corona discharge current at a preset magnitude of the high AC voltage.

14. The method according to claim 7, wherein in order to increase a concentration of H₂O₂, liquid hydrogen peroxide is recycled at least once to produce an air-liquid or vapor flow through the corona discharge zone.

## Patentansprüche

1. Vorrichtung zum Erzeugen von Wasserstoffperoxid, wobei die Vorrichtung umfasst:
ein Gehäuse (1) mit einem Einlass (12) zur Luft-Flüssigkeits- oder Dampfzufuhr und getrennten Auslässen (13, 7) für H₂O₂- und Luftabfluss,
erste und zweite Elektroden (6, 11), die angeordnet sind, um den Luft-Flüssigkeits- oder Dampffluss zu ermöglichen, und
eine Hochspannungswechselstromversorgung (14), die mit der ersten und zweiten Elektrode verbunden ist und konfiguriert ist, um zwischen den Elektroden eine Koronaentladungszone mit einem geraden Segment zu erzeugen;
wobei eine der ersten und zweiten Elektroden die Richtung des Luft-Flüssigkeits- oder Dampfflusses innerhalb des geraden Segments der Koronaentladungszone ändert; **dadurch gekennzeichnet, dass**:
die erste Elektrode (6), die die Richtung des Luft-Flüssigkeits- oder Dampfflusses ändert, an einen Hohlrotor (9) gekoppelt ist,
die zweite Elektrode (11) von einem Isolator (10) umgeben ist und innerhalb eines Hohlraums des Hohlrotors angeordnet ist, und
der H₂O₂-Auslass (13) im unteren Teil des Gehäuses (1) liegt.

2. Vorrichtung nach Anspruch 1, wobei mindestens ein Teil des Rotors aus einem elektrisch leitenden Material hergestellt ist.

3. Vorrichtung nach Anspruch 2, wobei die Hochspannungswechselstromzufuhr (14) mit dem Rotor (9) über eine kapazitive Kopplung (5, 6) verbunden ist.

4. Vorrichtung nach einem der Ansprüche 1 bis 3, wobei der Rotor (9) konfiguriert ist, um relativ zur zweiten Elektrode (11) zu rotieren.

5. Vorrichtung nach einem der Ansprüche 1 bis 4, wobei ein Innendurchmesser (D1) des Rotors (9) von einem Außendurchmesser des Isolators (D2) um einen vorbestimmten Abstand versetzt ist, dessen Länge gewählt ist, um einen erforderlichen Koronaentladungsstrom bei einer voreingestellten Größe des Hochspannungswechselstroms abzuleiten.

6. Vorrichtung nach Anspruch 1, wobei der Luftauslass und der Luft-Flüssigkeits- oder Dampfzuflusseinlass durch einen Bypass-Kanal verbunden sind.

7. Verfahren zum Erzeugen von Wasserstoffperoxid (H₂O₂) unter Verwendung der Vorrichtung nach einem der Ansprüche 1 bis 6, wobei das Verfahren umfasst:
Transportieren eines Luft-Flüssigkeits- oder Dampfflusses durch eine Koronaentladungszone (C) mit einem geraden Segment, die durch Anlegen eines Hochspannungswechselstroms (14) zwischen der ersten und der zweiten Elektrode (6, 11) erzeugt wird, wobei:
wobei der Winkel der Flussrichtung im Verlauf seines Übergangs in das gerade Segment der Koronaentladungszone verändert wird.

8. Verfahren nach Anspruch 7, wobei eine der Elektroden (11) innerhalb eines Hohlrotors (9) montiert ist, dessen Rotation eine Zentrifugalkraft bildet.

9. Verfahren nach Anspruch 8, wobei mindestens ein Teil des Rotors (9) aus einem elektrisch leitfähigen Material hergestellt ist.

10. Verfahren nach Anspruch 7, wobei der Hochspannungswechselstrom mit dem Rotor (9) über eine kapazitive Kopplung (5, 6) verbunden ist.

11. Verfahren nach Anspruch 7, wobei die zweite Elektrode (11) von einem Isolator (10) umgeben ist und innerhalb eines Hohlraums des Hohlrotors angeordnet ist.

12. Verfahren nach Anspruch 11, wobei der Rotor (9) relativ zur zweiten Elektrode (11) rotiert.

13. Verfahren nach Anspruch 7, wobei ein Innendurchmesser (D1) des Hohlrotors von einem Außendurchmesser (D2) der isolierenden Beschichtung der zweiten Elektrode um einen vorbestimmten Abstand versetzt ist, dessen Länge gewählt ist, um einen erforderlichen Koronaentladungsstrom bei einer voreingestellten Größe des Hochspannungswechselstroms abzuleiten.

14. Verfahren nach Anspruch 7, wobei, um eine H₂O₂-Konzentration zu erhöhen, flüssiges Wasserstoffperoxid mindestens ein Mal recycelt wird, um einen Luft-Flüssigkeits- oder Dampffluss durch die Koronaentladungszone zu erzeugen.

## Revendications

1. Dispositif pour produire du peroxyde d'hydrogène, le dispositif comprenant :
un logement (1) ayant une entrée (12) pour l'apport d'air-liquide ou de vapeur et des sorties séparées (13, 7) pour l'écoulement de H₂O₂ et de l'air,
des première et seconde électrodes (6, 11) positionnées pour permettre l'écoulement de l'air-liquide ou de la vapeur, et
une alimentation de tension AC élevée (14) connectée aux première et seconde électrodes et configurée pour produire entre les électrodes une zone de déchargement de couronne ayant un segment linéaire ;
où une des première et seconde électrodes change la direction de l'écoulement d'air-liquide ou de vapeur dans le segment linéaire de la zone de déchargement de couronne ;
**caractérisé en ce que** :
la première électrode (6), qui change la direction de l'écoulement d'air-liquide ou de vapeur, est couplée à un rotor creux (9),
la seconde électrode (11) est entourée par un isolateur (10) et est disposée dans une cavité du rotor creux, et
la sortie de H₂O₂ (13) est située dans la partie inférieure du logement (1).

2. Dispositif selon la revendication 1, dans lequel au moins une partie du rotor est constituée d'un matériau électriquement conducteur.

3. Dispositif selon la revendication 2, dans lequel l'alimentation de tension AC élevée (14) est connectée au rotor (9) via un couplage capacitif (5, 6).

4. Dispositif selon l'une quelconque des revendications 1 à 3, dans lequel le rotor (9) est configuré pour tourner relativement à la seconde électrode (11).

5. Dispositif selon l'une quelconque des revendications 1 à 4, dans lequel un diamètre interne (D1) du rotor (9) est déplacé d'un diamètre externe de l'isolateur (D2) par un espace libre prédéterminé, dont une longueur est choisie pour dériver un courant de décharge de couronne requis à une amplitude préréglée de la tension AC élevée.

6. Dispositif selon la revendication 1, dans lequel la sortie d'air et l'entrée d'écoulement d'air-liquide ou de vapeur sont connectées par un canal de découplage.

7. Procédé de production de peroxyde d'hydrogène (H₂O₂) utilisant le dispositif selon l'une quelconque des revendications 1 à 6, le procédé comprenant :
le transport de l'écoulement d'air-liquide ou de vapeur à travers une zone de déchargement de couronne (C) ayant un segment linéaire produit en appliquant une tension AC élevée (14) entre les première et seconde électrodes (6, 11), où :
l'angle de la direction d'écoulement est changé au cours de son passage dans le segment linéaire de la zone de déchargement de couronne.

8. Procédé selon la revendication 7, dans lequel une des électrodes (11) est montée dans un rotor creux (9), dont la rotation forme une force centrifuge.

9. Procédé selon la revendication 8, dans lequel au moins une partie du rotor (9) est constituée d'un matériau électriquement conducteur.

10. Procédé selon la revendication 7, dans lequel la tension AC élevée est connectée au rotor (9) via un couplage capacitif (5, 6).

11. Procédé selon la revendication 7, dans lequel la seconde électrode (11) est entourée par un isolateur (10) et positionnée à l'intérieur d'une cavité du rotor creux.

12. Procédé selon la revendication 11, dans lequel le rotor (9) est tourné relativement à la seconde électrode (11).

13. Procédé selon la revendication 7, dans lequel un diamètre interne (D1) du rotor creux est déplacé d'un diamètre externe (D2) du revêtement isolateur de la seconde électrode par un espace libre prédéterminé, dont une longueur est choisie pour dériver un courant de déchargement de couronne requis à une amplitude préréglée de la tension AC élevée.

14. Procédé selon la revendication 7, dans lequel pour augmenter une concentration de H₂O₂, du peroxyde d'hydrogène liquide est recyclé au moins une fois pour produire un écoulement d'air-liquide ou de vapeur à travers la zone de déchargement de couronne.
